# EUROPEAN PATENT SPECIFICATION

(11) **EP 1 633 837 B1**
(45) Date of publication and mention of the grant of the patent: **18.02.2009**
(21) Application number: 04739899.5
(22) Date of filing: 15.06.2004
(51) Int. Cl.: C11D 1/825

(54) **Emulsion**
Emulsion
Emulsion

(30) Priority: 16.06.2003 GB 0313830
(43) Date of publication of application: 15.03.2006
(73) Proprietor: Uniqema B.V., 2802 AA Gouda (NL)
(72) Inventor: GROENHOF, Franciscus, Johannes, NL-3981 VB Bunnik (NL)
(74) Representative: Humphries, Martyn
(86) International application number: PCT/EP2004/006429
(87) International publication number: WO 2004/111168

(56) References cited:
- US-A- 6 096 325
- US-A1- 2002 065 328
- US-A1- 2003 105 169
- UNIQEMA: "Formulation data for W/O/W sunscreen cream"[Online] 2002, XP002297470 Retrieved from the Internet: URL:http://www.uniqema.com/Uniqema_Product Catalogue/ProdCat/default.asp?Region=&PAGE 1=http://www.uniqema.com/Uniqema_ProductCa talogue/ProdCat/Formulations.asp?Region=&M oveTo=116> [retrieved on 2004-09-22]

## Description

### Field of Invention

The present invention relates to an emulsion formed using a surfactant composition, and in particular to a sprayable personal care or cosmetic product formed from the emulsion.

### Background

A wide range of surfactant compositions are known, which have been used for many purposes including stabilising oil water interfaces, particularly water in oil and oil in water emulsions and dispersions. These emulsions can be used in personal care or cosmetic products such as creams and milks, and desirably have a number of properties in combination, e.g. stability in manufacture, formulation, storage and use; a viscosity appropriate to the end use; and preferably a desirable body and good skin feel. Good body and skin feel are usually assessed subjectively, and can be difficult to obtain in combination with all the other desired properties. In particular, it can be difficult to achieve the required stability, especially in combination with good body and skin feel, in an emulsion that is suitable for spraying, such as in a sprayable sunscreen formulation.

The present invention is based on our discovery that certain combinations of surfactants can provide good emulsion stabilisation at low shear viscosity values, which enables the composition to be applied as a spray in use, and preferably also exhibit acceptable or even significantly improved body or skin feel.

US-6096325-A is directed to an oil in water skin care composition containing a silicone component, a liquid crystals forming emulsifier and an ethoxylated polyol fatty acid ester.

### Summary of the Invention

We have now surprisingly discovered an emulsion which overcomes or significantly reduces at least one of the aforementioned problems.

Accordingly, the present invention provides an oil in water emulsion comprising an emulsifier system for the oil which comprises at least one branched alkoxylated non-ionic surfactant and 0.5 to 10% by weight of at least one surfactant capable of forming liquid crystals in water, wherein the water phase comprises liquid crystals.

The invention also provides a personal care or cosmetic product comprising an oil in water emulsion comprising an emulsifier system for the oil which comprises at least one branched alkoxylated non-ionic surfactant and 0.5 to 10% by weight of the emulsion of at least one surfactant capable of forming liquid crystals in water, wherein the water phase comprises liquid crystals.

The invention further provides a container comprising a spray nozzle and a sprayable personal care or cosmetic product comprising an oil in water emulsion comprising an emulsifier system for the oil which comprises at least one branched alkoxylated non-ionic surfactant and 0.5 to 10% by weight of the emulsion of at least one surfactant capable of forming liquid crystals in water, wherein the water phase comprises liquid crystals.

The invention still further provides the use of a surfactant composition comprising at least one branched alkoxylated non-ionic surfactant and at least one surfactant capable of forming liquid crystals in water to stabilize an oil in water emulsion, wherein the water phase comprises liquid crystals and the amount of surfactant capable of forming liquid crystals is 0.5 to 10% by weight of the emulsion.

The branched non-ionic surfactant used in the present invention comprises a branched hydrophobe moiety and a hydrophile moiety.

The hydrophobe moiety of the branched non-ionic surfactant may comprise an aliphatic, cycloaliphatic or aromatic group, but is preferably an aliphatic group, i.e. does not contain a cycloaliphatic group or an aromatic group. The hydrophobe moiety may be unsaturated, for example comprising one or more carbon double bonds, but is preferably saturated. The hydrophobe moiety preferably comprises a branched hydrocarbyl group, more preferably a branched alkyl group. The mean number of carbon atoms (on a molar basis) in the hydrophobe moiety is suitably in the range from 10 to 22, preferably 12 to 20, more preferably 13 to 19, particularly 14 to 18, and especially 15 to 17. As the numerical value for the number of carbon atoms is expressed as an average (i.e. mixtures may be employed), it may be non-integral.

The mean number of carbon atoms in the longest linear chain of the preferred branched alkyl group is preferably in the range from 7 to 15, more preferably 8 to 13, particularly 9 to 11, and especially 9.5 to 10.5. The mean number of branches in the branched alkyl group is suitably less than 5, preferably less than 4, more preferably in the range from 0.2 to 3, particularly 0.5 to 2, and especially 0.8 to 1.2. The branches (hereinafter referred to as side-branches) are preferably alkyl groups, more preferably linear, and particularly attached directly to a carbon atom of the longest linear chain. The mean number of carbon atoms in the preferred side-branched alkyl groups is suitably in the range from 1 to 10, preferably 2 to 9, more preferably 3 to 8, particularly 4 to 7, and especially 5 to 6. In a preferred embodiment of the invention, greater than 50%, more preferably greater than 70%, particularly greater than 90%, and especially greater than 95% and up to 100% of the side-branched groups are within the aforementioned preferred ranges for the number of carbon atoms.

In an alternative preferred embodiment of the invention, the mean number of carbon atoms in the alkyl side branches of the branched alkyl group is less than 5, more preferably less than 3, particularly in the range from 1.05 to 2, and especially 1.1 to 1.5, i.e. the side branches are predominantly methyl groups. In addition, preferably greater than 50%, more preferably greater than 60%, particularly in the range from 70 to 97%, and especially 80 to 93% of the side-branched groups are methyl groups. Further, preferably greater than 30%, more preferably greater than 40%, particularly in the range from 45 to 90%, and especially 50 to 80% of the side branches contain single methyl groups. In a preferred embodiment, the branched alkyl group is an isostearyl group, preferably derived from isostearic acid.

The hydrophile moiety of the branched non-ionic surfactant preferably comprises a polyoxyalkylene group. The polyoxyalkylene group is suitably of the formula (CₘH₂ₘO)ₙ where m is 2, 3 or 4, preferably 2 or 3, and more preferably 2, i.e. an oxyethylene group. The polyoxyalkylene chain may be substantially or wholly of oxyethylene residues, or substantially or wholly of oxypropylene residues, or it may include both oxyethylene and oxypropylene residues to give a random or block copolymer chain. The chain is preferably a homopolymeric polyoxyethylene chain. The mean value of n is suitably in the range from 5 to 40, preferably 10 to 30, more preferably 15 to 25, and particularly 18 to 22, especially 19 to 21. As these are average values, n may be non-integral. The aforementioned values are particularly preferred when the polyoxyalkylene chain is wholly a polyoxyethylene chain. Where the chain is a block or random copolymer of oxyethylene and oxypropylene residues, the chain length chosen will typically correspond to the above ranges, but will depend upon the proportion of oxyethylene and oxypropylene residues in the chain. In copolymer chains usually oxyethylene residues will provide at least 60 mole% of the total oxyalkylene residues. Oxybutylene residues can be included in the chain, but when present these will usually be present as a minor component of the chain e.g. up to about 20 mole% of the total polyoxyalkylene chain.

The hydrophobe moiety and hydrophile, preferably polyoxyalkylene-containing, moiety of the branched non-ionic surfactant used in the present invention, are suitably connected through a linking group, preferably through the residue of a linking group having a reactive hydrogen atom to an oligomeric or polymeric chain of alkylene oxide residues. The linking group may be an oxygen atom (hydroxyl group residue); a carboxyl group (fatty acid or ester group residue); an amino group (amine group residue); or a carboxyamido (carboxylic amide group residue). In a preferred embodiment of the invention, the linking group is an oxygen atom.

Examples of suitable branched non-ionic alkoxylate surfactants include alcohol alkoxylates, of the formula R¹ - O - (CₘH₂ₘO)ₙ - H; a fatty acid alkoxylate of the formula R¹ - COO - (CₘH₂ₘO)ₙ - R² (plus co-products); a fatty amine alkoxylate of the formula R¹ - NR³ - (CₘH₂ₘO)ₙ - H; or a fatty amide alkoxylate of the formula R¹ - NR³ - (CₘH₂ₘO)ₙ - H, where each R¹ is independently a hydrophobe moiety as defined above; R² is a hydrogen atom or a-C₁ to C₆-alkyl group; and each R³ is independently a C₁ to C₆ alkyl-group or a group (CₘH₂ₘO)ₙ - H; in each (CₘH₂ₘO)ₙ, m and/or n is independently as defined above. In a preferred embodiment of the invention, the branched non-ionic surfactant is an alcohol alkoxylate, more preferably a primary alcohol alkoxylate, of the formula R¹ - O - -(CₘH₂ₘO)ₙ - H.

In a particularly preferred embodiment of the present invention, the branched non-ionic surfactant comprises at least one branched primary alcohol alkoxylate of the formula (I):

CH₃ - (CH₂)_{q} - [CH₃- (CH₂)ᵣ] - CH - (CH₂)ₚ - O - (CₘH₂ₘO)ₙ - H (I)

where
q and r are each independently from 0 to 13, preferably 1 to 15, more preferably 2 to 13; and p is 1 or 2;
such that q+r+p is in the range from 9 to 17, preferably 10 to 16, more preferably 11 to 15; and
(CₘH₂ₘO)ₙ is a polyoxyalkylene group as defined above, m and n are as defined above.

In compounds of the formula (I),
q and r are (i) preferably each independently at least 2, more preferably at least 4, and particularly at least 5, and/or (ii) preferably not more than 10, more preferably not more than 9, and particularly not more than 8,
p is preferably 1, and
the total number of carbon atoms in the branched alkyl group is preferably in the range from 12 to 20, more preferably 13 to 19, particularly 14 to 18, and especially 15 to 17 (corresponding to q+r+p preferably in the range from 9 to 17, more preferably 10 to 16, particularly 11 to 15, and especially 12 to 14).

The preferred primary alkoxylate compounds used in this invention can be made by alkoxylation of the corresponding branched primary alcohols under conventional alkoxylation conditions, typically under alkali catalysis, particularly alkoxide catalysis e.g. using NaOH or KOH to form alkoxide *in situ.* The branched primary alcohols can be substantially wholly branched alcohols preferably as made by the guerbet process e.g. 2-butyloctanol, 2-butyldecanol, 2-butyldodecanol, 2-hexyl-octanol, 2-hexyldecanol, 2-hexyldodecanol and 2-octyldecanol. In a particularly preferred embodiment, 2-hexyldecanol is used as the branched primary alcohol. Thus, polyoxyethylene isocetylether, preferably containing in the range from 18 to 22 ethylene oxide groups, is a particularly preferred branched non-ionic surfactant. A suitable material is available commercially under the trade mark Arlasolve 200 from Uniqema.

In an alternative embodiment, mixtures of branched primary alcohols with linear primary alcohols, containing similar numbers of carbon atoms can be employed, such as can be made by the oxo process starting with internal olefins under conditions where the double bond can migrate. Commercially available branched primary alcohols can be substantially pure or can include up to about 50% of substantially linear alcohols. In such mixed materials the proportion of branched material having branches at least two carbon atoms long is preferably at least about 30%, more preferably about 40%, and the proportion of branched material having branches at least three carbon atoms long is preferably at least about 20%, more preferably about 30%. On alkoxylation, such materials give mixtures of linear and branched alcohol alkoxylates.

The branched non-ionic surfactant suitably has a Hydrophile Lipophile Balance (HLB) value of greater than 10, preferably in the range from 11 to 20, more preferably 13 to 18, particularly 14 to 17, and especially 15 to 16.

The amount of branched non-ionic surfactant in a composition according to the present invention is suitably in the range from 5 to 95%, preferably 10 to 90%, more preferably 20 to 70%, particularly 30 to 55%, and especially 35 to 45%, by weight of the total amount of branched non-ionic surfactant and surfactant capable of forming liquid crystals in water, in the composition.

The surfactant capable of forming liquid crystals in water, which is used in the present invention, preferably forms liquid crystals, more preferably in emulsions, and particularly in oil in water emulsions. The liquid crystals which are formed are preferably lyotropic liquid crystals (i.e. both concentration and temperature dependant), more preferably lamellar phase liquid crystals, and particularly L alpha phase (neat) liquid crystals.

The surfactant capable of forming liquid crystals in water may be a polyol ester, an alkoxylated polyol ester, an acyl lactylate, and mixtures thereof. A polyol ester is preferred, particularly a polyol ester of a fatty acid or derivative thereof, i.e. preferably a non-alkoxylated species.

In a preferred embodiment, the polyol is glycerol, polyglycerol, pentaerythritol, a saccharide, and/or an anhydro-saccharide, and more preferably is an anhydro-saccharide, particularly sorbitan and/or mannitan, and especially sorbitan. The preferred fatty acid is suitably saturated, and the mean number of carbon atoms thereof is preferably in the range from 8 to 24, more preferably 12 to 22, particularly 16 to 20, and especially 17 to 19. Suitable fatty acids include lauric, myristic, palmitic, stearic, and/or behenic acid. Sorbitan stearate is particularly preferred. The preferred sorbitan ester is preferably used as the mono-ester, although minor amounts of the higher esters may also be present.

The preferred anhydro-saccharide ester suitably has a HLB value in the range from 3. to 10, preferably 3.5 to 8, more preferably 4 to 6, particularly 4.4 to 5, and especially 4.6 to 4.8.

In a particularly preferred embodiment of the present invention, the surfactant capable of forming liquid crystals in water comprises an anhydro-saccharide ester as defined above and an additional polyol ester derived from a saccharide (or non-anhydro-saccharide), more preferably a sugar, particularly sorbitol, mannitol, sucrose, fructose and/or glucose, and especially sucrose or sorbitol.

The saccharide ester suitably has a HLB value of greater than 10, preferably in the range from 12 to 24, more preferably 14 to 20, particularly 16 to 18, and especially 16.5 to 17.5.

The saccharide ester is preferably formed from a fatty acid component, or derivative thereof, containing a mean number of carbon atoms in the range from 8 to 20, more preferably 8 to 18, particularly 10 to 16, and especially 12 to 14. Suitable fatty acids include lauric, myristic, and/or palmitic acid, and/or naturally occurring mixtures of fatty acids, such as fatty acids derived from palm kernel oil or coconut oil.

When the saccharide is sucrose, coconut oil fatty acids are particularly preferred. The fatty acid mixture preferably comprises any one or more, and preferably all, of the following; (i) less than 10%, more preferably less than 5% by weight of C₈, (ii) less than 8%, more preferably less than 5% by weight of C₁₀,- (iii) in the range from 45 to 60%, more preferably 50 to 55% by weight of C₁₂, (iv) in the range from 15 to 25%, more preferably 17 to 21 % by weight of C₁₄, (v) less than 14%, more preferably less than 10% by weight of C₁₆, (vi) in the range from 5 to 20%, more preferably 10 to 15% by weight of C₁₈, (vii) less than 5%, more preferably less than 2% by weight of C₂₀ and/or (viii) less than 15%, more preferably less than 10% by weight of unsaturated fatty acids. The sucrose cocoate preferably has a relatively high proportion of mono-ester, more preferably of at least 50% particularly at least 65%, and especially at least 70% by weight.

When the saccharide is sorbitol, the fatty acid is preferably lauric acid. The sorbitol laurate preferably comprises a mixture of mono- and di-esters. The concentration of (i) monoesters is preferably at least 50%, more preferably at least 70%, particularly at least 80%, and especially at least 85% by weight, and (ii) diesters is preferably less than 50%, more preferably less than 30%, particularly less than 20%, and especially less than 15% by weight.

In a preferred embodiment of the invention, the surfactant capable of forming liquid crystals in water comprises a mixture of an anhydro-saccharide, preferably sorbitan, ester and a saccharide, preferably sucrose or sorbitol, ester. Particularly preferred blends are mixtures of (i) sorbitan stearate and sucrose cocoate, and (ii) sorbitan stearate and sorbitol laurate. These materials are available commercially under the trade mark Arlatone 2121 and Arlatone LC from Uniqema.

The ratio by weight of anhydro-saccharide ester to saccharide ester is suitably in the range from 0.5 to 100:1, preferably 2 to 50:1, more preferably 4 to 20:1, particularly 6 to 12:1, and especially approximately 9:1 by weight.

The surfactant (or mixture of surfactants) capable of forming liquid crystals in water suitably has a HLB value in the range from 2 to 10, preferably 3 to 9, more preferably 4 to 8, particularly 5 to 7, and especially 5.5 to 6.5.

The amount of surfactant capable of forming liquid crystals in water in a composition according to the present invention is suitably in the range from 5 to 95%, preferably 10 to 90%, more preferably 30 to 80%, particularly 45 to 70%, and especially 55 to 65%, by weight of the total amount of branched non-ionic surfactant and surfactant capable of forming liquid crystals in water, in the composition.

The ratio of branched non-ionic surfactant to surfactant capable of forming liquid crystals in water present in a composition according to the present invention is suitably in the range from 0.05 to 20:1, preferably 0.1 to 10:1, more preferably 0.4 to 4:1, particularly 0.8 to 2.5:1, and especially 1 to 1.5:1 by weight.

The surfactant composition according to the present invention suitably has a Hydrophile Lipophile Balance (HLB) value in the range from 6 to 14, preferably 7 to 13, more preferably 8 to 12, particularly 9 to 11.5, and especially 10 to 11.

The surfactant composition defined herein is suitable for use in forming emulsions (and dispersions), i.e. as the, or as part of the, emulsifier system, such as water in oil emulsions, particularly oil in water emulsions, and especially for use in personal care or cosmetic products.

The oil phase of the emulsion according to the present invention will preferably mainly be an emollient oil of the type used in personal care or cosmetic products. The emollient can and usually will be an oily material which is preferably liquid at ambient temperature. Alternatively, it can be solid at ambient temperature, in which case in bulk it will usually be a waxy solid, provided it is liquid at an elevated temperature at which it can be included in and emulsified in the composition. The manufacture of the composition preferably uses temperatures up to 100°C, more preferably about 80°C, and therefore such solid emollients will preferably have melting temperatures of less than 100°C, and more preferably less than 70°C. Suitable normally liquid emollient oils include non-polar oils, for example mineral or paraffin, especially isoparaffin, oils, such as that sold by Uniqema as Arlamol (trade mark) HD; or medium polarity oils, for example vegetable ester oils such as jojoba oil, vegetable glyceride oils, animal glyceride oils, such as that sold by Uniqema as Estol (trade mark) 3603 (caprylic/capric triglyceride), synthetic oils, for example synthetic ester oils, such as isopropyl palmitate and those sold by Uniqema as Estol 1512 and Arlamol DOA, ether oils, particularly of two fatty e.g. C8 to C18 alkyl residues, such as that sold by Cognis as Cetiol OE (dicaprylether), guerbet alcohols such as that sold by Cognis as Eutanol G (octyl dodecanol), or silicone oils, such as dimethicione oil such as those sold by Dow Corning as DC200, cyclomethicone oil, or silicones having polyoxyalkylene side chains to improve their hydrophilicity; or highly polar oils including alkoxylate emollients for example fatty alcohol propoxylates such as that sold by Uniqema as Arlamol E (propoxylated stearyl alcohol). Suitable emollient materials that can be solid at ambient temperature but liquid at temperatures typically used to make the compositions of this invention include jojoba wax, tallow and coconut wax/oil. When non-polar oils are used it may be desirable to use relatively high concentrations of surfactant, particularly high HLB surfactant, in order to achieve suitably satisfactory emulsification, particularly to obtain small oil droplets.

Mixtures of emollients can and often will be used, and in some cases solid emollients may dissolve wholly or partly in liquid emollients, or in combination the freezing point of the mixture is suitably low. Where the emollient composition is a solid (such as fatty alcohols) at ambient temperature, the resulting dispersion may technically not be an emulsion (although in most cases the precise phase of the oily disperse phase cannot readily be determined) but such dispersions behave as if they were true emulsions and the term emulsion is used herein to include such compositions.
The concentration of the oil phase may vary widely. The amount of the oil phase in the emulsion is suitably in the range from 1 to 90%, preferably 2 to 60%, more preferably 5 to 40%, particularly 10 to 30%, and especially 15 to 25% by weight of the total composition.

The amount of water present in the emulsion is suitably in the range from 10 to 95%, preferably 30 to 85%, more preferably 40 to 80%, particularly 50 to 75%, and especially 60 to 70% by weight of the total composition.

The amount of branched non-ionic surfactant in an emulsion or personal care or cosmetic product according to the present invention is suitably in the range from 0.1 to 10%, preferably 0.5 to 5%, more preferably 1 to 3.5%, particularly 1.5 to 2.5%, and especially 1.8 to 2.2%, by weight of the emulsion.

The amount of surfactant capable of forming liquid crystals in water in an emulsion or personal care or cosmetic product according to the present invention is suitably in the range from 0.5 to 10%, preferably 0.5 to 7%, more preferably 1 to 5%, particularly 2 to 4%, and especially 2.5 to 3.5%, by weight of the emulsion.

The emulsions according to the present invention may also contain other additional surfactant materials which form part of the emulsifier system. Other suitable surfactants include relatively hydrophilic surfactants, e.g. having a HLB value of greater than 10, preferably greater than 12, and relatively hydrophobic surfactants e.g. having a HLB value of less than 10, preferably less than 8. Relatively hydrophilic surfactants include alkoxylate surfactants with an average in the range from about 10 to about 100 alkylene oxide, particularly ethylene oxide residues; and relatively hydrophobic surfactants include alkoxylate surfactants preferably with an average in the range from about 3 to about 10 alkylene oxide, particularly ethylene oxide residues.

Personal care or cosmetic emulsions can be divided by viscosity into milks and lotions, which preferably have a low shear viscosity (measured at shear rates of about 0.1 to 10 s⁻¹ as is typically used in Brookfield viscometers) of up to 10,000 mPa.s, and creams which preferably have a low shear viscosity of more than 10,000 mPa.s. Milks and lotions preferably have a low shear viscosity in the range from 100 to 10,000, more preferably 200 to 5,000, and particularly 300 to 1,000 mPa.s.

Creams preferably have a low shear viscosity of at least 20,000, more preferably in the range from 30,000 to 80,000, and particularly 40,000 to 70,000 mPa.s, although even higher viscosities e.g. up to about 10⁶ mPa.s, may also be used.

Emulsions according to the present invention are particularly suitable for use as a sprayable personal care or cosmetic product. Such emulsions suitably have a low shear viscosity of up to 15,000, preferably up to 10,000, more preferably in the range from 100 to 5,000, particularly 200 to 3,000, and especially 300 to 1,000 mPa.s. It is a particularly surprising feature of the present invention that sprayable emulsions having a low shear viscosity within the aforementioned ranges can be produced which also exhibit good stability and/or good skin feel.

The emulsions according to the present invention are stable, measured as described herein, preferably for greater than one month, more preferably greater than two months, particularly greater than three months, and especially greater than four months at ambient temperature (23°C). The stability at elevated temperatures is particularly important, and therefore the emulsion is stable, measured as described herein, suitably for greater than one week, preferably greater than two weeks, more preferably greater than 1 month, particularly greater than two months, and especially greater than three months at 40°C, and preferably also at 50°C. In a particularly preferred embodiment, the liquid crystals created during emulsion formation are substantially maintained during the aforementioned time and temperature testing regimes.

The emulsions of the invention can be made by generally conventional emulsification and mixing methods. Typical methods for producing oil in water emulsions include direct emulsification by dispersing the surfactant capable of forming liquid crystals in water in the water phase, dispersing the branched non-ionic surfactant in the oil phase, and then mixing in and emulsifying the oil in the aqueous continuous phase. It is desirable to either heat the aqueous phase usually above about 60°C, e.g. to about 80 to 85°C, or to subject the aqueous phase to high intensity mixing at lower, e.g. about ambient, temperature. Vigorous mixing and the use of moderately elevated temperatures can be combined if desired. The heating and/or high intensity mixing can be carried out before, during or after addition of the oil phase.

The emulsions can also be made by inverse emulsification methods, whereby the surfactant capable of forming liquid crystals is added to the oil phase, the branched non-ionic surfactant is added to the aqueous phase, which is then added and mixed into the oil phase to form a water in oil emulsion. Aqueous phase addition is continued until the system-inverts to form an oil in water emulsion. Plainly a substantial amount of aqueous phase will generally be needed to effect inversion and so this method is not likely to be used for high oil phase content emulsions. Vigorous mixing and the use of moderately elevated temperatures can be combined if desired. The heating and/or high intensity mixing can be carried out during or after addition of the aqueous phase and before during or after inversion.

The emulsions according to the present invention also surprisingly possess very good body and skin feel, exhibiting good spreadability, a light skin feel and/or a light afterfeel.

Many other components may be included in the emulsions to make personal care or cosmetic compositions or products. These components can be oil soluble, water soluble or non-soluble. Examples of such materials include:
(i) preservatives such as those based on parabens (alkyl esters of 4-hydroxybenzoic acid), phenoxyethanol, substituted ureas and hydantoin derivatives e.g. those sold commercially under the trade names Germaben II Nipaguard BPX and Nipaguard DMDMH, when used usually in a concentration of from 0.5 to 2% by weight of the total composition;
(ii) perfumes, when used typically at a concentration of from 0.1 to 10% more usually up to about 5% and particularly up to about 2% by weight of the total composition;
(iii) humectants or solvents such as alcohols, polyols such as glycerol and polyethylene glycols, when used typically at a concentration of from 1 to 10% by weight of the total composition;
(iv) sunfilter or sunscreen materials including organic sunscreens and/or inorganic sunscreens including those based on titanium dioxide or zinc oxide; when used typically at from 0.1% to 5% by weight of the total;
(v) alpha hydroxy acids such as glycolic, citric, lactic, malic, tartaric acids and their esters; self-tanning agents such as dihydroxyacetone;
(vi) antimicrobial, particularly anti-acne components such as salicylic acid;
(vii) vitamins and their precursors including: (a) Vitamin A, e.g. as retinyl palmitate and other tretinoin precursor molecules, (b) Vitamin B, e.g. as panthenol and its derivatives, (c) Vitamin C, e.g. as ascorbic acid and its derivatives, (d) Vitamin E, e.g. as tocopheryl acetate, (e) Vitamin F e.g. as polyunsaturated fatty acid esters such as gamma-linolenic acid esters;
(viii) skin care agents such as ceramides either as natural materials or functional mimics of natural ceramides;
(ix) natural phospholipids, eg lecithin;
(x) vesicle-containing formulations;
(xi) germanium-containing compounds for example that sold by Uniqema as Arlamol GEO;
(xii) botanical extracts with beneficial skin care properties;
(xiii) skin whiteners such as Arlatone Dioic DCA (trade mark) sold by Uniqema, kojic acid, arbutin and similar materials;
(xiv) skin repair compounds actives such as Allantoin and similar series;
(xv) caffeine and similar compounds;
(xvi) cooling additives such as menthol or camphor;
(xvii) insect repellents such as N,N-diethyl-3-methylbenzamide (DEET) and citrus or eucalyptus oils;
(xviii) essential oils; and
(xix) pigments, including microfine pigments, particularly oxides and silicates, e.g. iron oxide, particularly coated iron oxides, and/or titanium dioxide, and ceramic materials such as boron nitride, or other solid components, such as are used in make up and cosmetics, to give suspoemulsions, typically used in an amount of from about 1 to about 15%, but usually at least about 5% and particularly about 10%.

A surprising feature of the present invention is that stable emulsions can be obtained, which can still be sprayable, when containing high concentrations of solid materials, for example of particulate titanium dioxide and/or zinc oxide, preferably of up to 20%, more preferably up to 15%, particularly in the range from 1 to 10%, and especially 2 to 6% by weight of the total composition.

A further surprising feature is that stable emulsions can be obtained containing high concentrations of alcohols, for example ethanol, preferably of up to 20%, more preferably up to 15%, particularly in the range from 2 to 10%, and especially 4 to 8% by weight of the total composition.

The compositions according to the present invention are particularly suitable for use as sprayable personal care or cosmetic products, such as sunscreens, and on wipes. The advantages of a sprayable composition include coverage of a large surface area in a short period of time, and the cooling effect that can.be obtained by evaporation of solvent after the composition has been sprayed onto the surface of the skin.

A preferred application is as a sunscreen, particularly a sprayable sunscreen. The sunscreen may contain one or more organic sunscreens and/or inorganic sunscreens such as metal oxides, but preferably comprises at least one particulate titanium dioxide and/or zinc oxide, particularly included in the composition in the form of an aqueous and/or organic, preferably oil, dispersion available commercially from Uniqema under the trade marks Tioveil and Solaveil Clarus (both titanium dioxide) and Spectraveil (zinc oxide). In additon,organic sunscreens may be used together with the preferred metal oxide sunscreens, and include p-methoxy cinnamic acid esters, salicylic acid esters, p-amino benzoic acid esters, non-sulphonated benzophenone derivatives, derivatives of dibenzoyl methane and esters of 2-cyanoacrylic acid. Specific examples of useful organic sunscreens include benzophenone-1, benzophenone-2, benzophenone-3, benzophenone-6, benzophenone-8, benzophenone-12, isopropyl dibenzoyl methane, butyl methoxy dibenzoyl methane, ethyl dihydroxypropyl PABA, glyceryl PABA, octyl dimethyl PABA, octyl methoxycinnamate, homosalate, octyl salicylate, octyl triazone, octocrylene, etocrylene, menthyl anthranilate, 4-methylbenzylidene camphor, benzophenone 4, and phenyl benzimidazole sulphonic acid.

A particularly surprising feature of the present invention is that a sunscreen product described herein can be obtained with improved Sun Protection Factor (SPF) (measured as herein described), suitably having a SPF value of greater than 10, preferably greater than 15, more preferably greater than 20, particularly in the range from 25 to 50, and especially 30 to 40.

In this specification the following test methods have been used.

### Test methods:

(i) Viscosity was measured at 23°C with a Brookfield LVT viscometer using an appropriate spindle (LV1, LV2, LV3, or LV4 - depending on the viscosity of the emulsion being tested) at 6 rpm (0.1 Hz), 1 day after making the emulsions and results are quoted in mPa.s.
(ii) Stability was assessed by observing the emulsions after storage at ambient temperature (23°C), cold at 5°C or under elevated temperature storage at 40°C and 50°C. Measuring storage stability at 50°C is a very severe test. The composition is stable if no visible separation of the emulsion occurs. The stability of the liquid crystals in the emulsion was also assessed by observing under a microscope using polarized light.
(iii) Appearance was assessed visually and by skin feel using the following ratings:
   1 - very good has appearance highly suitable for end use and good skin feel with good shear thinning
   2 - good has appearance suitable for end use and moderate skin feel with some shear thinning
   3 - acceptable appearance and skin feel are acceptable for end use
   4 - poor appearance somewhat slimy and/or stringy and skin feel not particularly good
   5 - very poor appearance very slimy and stringy and poor skin feel
(iv) The fluidity of the product emulsions was assessed visually and the comments are descriptive in relation to the intended product type (milk, cream etc.).
(v) The Sun Protection Factor (SPF) of a sunscreen formulation was determined using the *in vitro* method of Diffey and Robson, J. Soc. Cosmet. Chem. Vol. 40, pp 127-133,1989.

The invention is illustrated by the following non-limiting examples.

### Examples

### Example 1

| **A.** | %w/w |
|---|---|
| ARLASOLVE 200 (trade mark, ex Uniqema) | 2.0 |
| ESTOL 3609 (trade mark, ex Uniqema) | 29.0 |
| ARLAMOL E (trade mark, ex Uniqema) | 29.0 |

| **B.** | |
|---|---|
| Water | 25.8 |
| ARLATONE 2121 (trade mark, ex Uniqema) | 3.0 |
| Keltrol F | 0.034 |
| PRICERINE 9091 (trade mark, ex Uniqema) | 1.2 |

| **C.** | |
|---|---|
| Ethanol | 10.0 |

### Procedure

1. Disperse Keltrol F in Phase B water, and add Pricerine 9091.
2. Add remaining Phase B ingredients.
3. Heat Phases B to 80°C, and leave for 20 minutes to allow Arlatone 2121 to swell.
4. Heat Phase A items to 80°C.
5. Slowly add A to B with intensive stirring at 500 rpm.
6. Homonegise at high shear for 1 minute at 9500 rpm using an Ultra Turrax T25 homogeniser.
7. Cool with stirring at 200 to 300 rpm to 40°C, add C with high shear mixing at 9500 rpm until homogeneous.
8. Cool to RT with stirring at 200 to 300 rpm.

The emulsion was subjected to the test methods described herein and exhibited the following properties;
(i) viscosity was 4900 mPa.s,
(ii) stable for at least 4 weeks at 5, 23, 40 and 50°C.
(iii) appearance and skin feel rating was 2, and
(iv) low viscosity and could be poured from a bottle, and sprayed from a pumpspray bottle.

### Example 2

| A. | %w/w |
|---|---|
| ARLASOLVE 200 (trade mark, ex Uniqema) | 2.0 |
| ESTOL 3609 (trade mark, ex Uniqema) | 14.0 |
| ARLAMOL E (trade mark, ex Uniqema) | 14.0 |

| **B.** | |
|---|---|
| Water | 62.3 |
| ARLATONE 2121 (trade mark, ex Uniqema) | 3.0 |
| STRUCTURE ZEA (trade mark, ex National Starch) | 1.0 |
| PRICERINE 9091 (trade mark, ex Uniqema) | 3.0 |

| **C.** | |
|---|---|
| Nipaguard BPX | 0.07 |

### Procedure

1. Disperse Structure Zea in Phase B water, and add Pricerine 9091.
2. Add remaining Phase B ingredients.
3. Heat Phases B to 80°C, and leave for 20 minutes to allow Arlatone 2121 to swell.
4. Heat Phase A items to 80°C.
5. Slowly add A to B with intensive stirring at 500 rpm.
6. Homonegise at high shear for 1 minute at 9500 rpm using an Ultra Turrax T25 homogeniser.
7. Cool with stirring at 200 to 300 rpm to 40°C, add C.
8. Cool to RT with stirring at 200 to 300 rpm.

The emulsion was subjected to the test methods described herein and exhibited the following properties;
(i) viscosity was 1120 mPa.s,
(ii) stable for at least 4 weeks at 5, 23, 40 and 50°C.
(iii) appearance and skin feel rating was 1, and
(iv) very low viscosity and could be poured from a bottle, and sprayed from a pumpspray bottle.

### Example 3

| **A.** | %w/w |
|---|---|
| ARLASOLVE 200 (trade mark, ex Uniqema) | 2.0 |
| ARLAMOL HD (trade mark, ex Uniqema) | 2.0 |
| Antaron V-220 | 2.0 |
| Vitamin E Acetate | 0.5 |
| TIOVEIL 50 FIN (trade mark, ex Uniqema) | 14.0 |
| Parsol MCX | 7.5 |

| **B.** | |
|---|---|
| Water | 63.4 |
| ARLATONE 2121 (trade mark, ex Uniqema) | 3.0 |
| STRUCTURE ZEA (trade mark, ex National Starch) | 1.0 |
| PRICERINE 9091 (trade mark, ex Uniqema) | 3.0 |
| Disodium EDTA | 0.1 |

| **C.** | |
|---|---|
| Panthenol 75 L | 0.8 |
| Nipaguard BPX | 0.7 |

### Procedure

1. Disperse Structure Zea in Phase B water, and add Pricerine 9091.
2. Add remaining Phase B ingredients.
3. Heat Phases B to 80°C, and leave for 20 minutes to allow Arlatone 2121 to swell.
4. Heat Phase A items without Tioveil 50 FIN to 80°C, at 80'C add Tioveil 50 FIN.
5. Slowly add A to B with intensive stirring at 500 rpm.
6. Homonegise at high shear for 1 minute at 9500 rpm using an Ultra Turrax T25 homogeniser.
7. Cool with stirring at 200 to 300 rpm to 40°C, add C.
8. Cool to RT with stirring at 200 to 300 rpm.

The emulsion was subjected to the test methods described herein and exhibited the following properties;
(i) viscosity was 750 mPa.s,
(ii) stable for at least 4 weeks at 5, 23, 40 and 50°C.
(iii) appearance and skin feel rating was 1,
(iv) very low viscosity/very fluid and could be poured from a bottle, and sprayed from a pumpspray bottle, and
(v) SPF was 30.5.

The above examples illustrate the improved properties of a surfactant composition and emulsion according to the present invention.

## Claims

1. An oil in water emulsion comprising an emulsifier system for the oil which comprises at least one branched alkoxylated non-ionic surfactant and 0.5 to 10% by weight of at least one surfactant capable of forming liquid crystals in water, wherein the water phase comprises liquid crystals.

2. An emulsion according to claim 1 wherein the branched alkoxylated non-ionic surfactant comprises a branched alkyl group comprising in the range from 12 to 20 carbon atoms.

3. An emulsion according to claim 2 wherein the branched alkyl group comprises In the range from 0.2 to 3 side-branches.

4. An emulsion according to claim 3 wherein the side branched groups are alkyl groups comprising in the range from 2 to 9 carbon atoms.

5. An emulsion according to any one of the preceding claims wherein the alkoxylate group is a homopolymeric polyoxyethylene chain containing in the range from 15 to 25 ethylene oxide residues.

6. An emulsion according to any one of the preceding claims wherein the branched alkoxylated non-ionic surfactant comprises a primary alcohol alkoxylate.

7. An emulsion according to claim 7 wherein the primary alcohol alkoxylate is of the formula:
CH₃ - (CH₂)_{q} - [CH₃- (CH₂)ᵣ] - CH - (CH₂)ₚ - O - (CₘH₂ₘO)ₙ - H (I)
wherein
q and r are each independence from 0 to 13, preferably 1 to 15, more preferably 2 to 13; and p is 1 or 2;
such that q+r+p is in the range from 9 to 17, preferably 10 to 16, more preferably 11 to 15; and
(CₘH₂ₘO)ₙ is a polyoxyalkylene group where m is 2, 3 or 4, and n is in the range from 5 to 40.

8. An emulsion according to any one of the preceding claims comprising 0.5 to 10% by weight of the branched alkoxylated non-ionic surfactant.

9. An emulsion according to any one of the preceding claims wherein the surfactant capable of forming liquid crystals in water comprises a non-alkoxylated polyol ester.

10. An emulsion according to claim 9 wherein the polyol is an anhydro-saccharide and/or the ester is derived from a fatty acid comprising in the range from 12 to 22 carbon atoms.

11. An emulsion according to either one of claims 10 and 11 wherein the surfactant capable of forming liquid crystals in water additionally comprises a polyol ester derived from a saccharide.

12. An emulsion according to claim 11 wherein the saccharide is sucrose or sorbitol, and/or the ester is derived from a fatty acid comprising in the range from 8 to 18 carbon atoms,

13. An emulsion according to any one of the preceding claims wherein the surfactant capable of forming liquid crystals in water comprises a mixture of a sorbitan ester and a sucrose ester or a sorbitol ester.

14. An emulsion according to claim 13 wherein the surfactant capable of forming liquid crystals in water comprises a mixture of sorbitan stearate and sucrose cocoate or sorbitol laurate.

15. An emulsion according to any one of the preceding claims wherein the HLB value of (i) the branched alkoxylated non-ionic surfactant is in the range from 13 to 18, (ii) the surfactant capable of forming liquid crystals in water is in the range from 4 to 8, and (iii) the total surfactant composition is in the range from 8 to 12.

16. A personal care or cosmetic product comprising an oil in water emulsion comprising an emulsifier system for the oil which comprises at least one branched alkoxylated non-ionic surfactant and 0.5 to 10% by weight of the emulsion of at least one surfactant capable of forming liquid crystals in water, wherein the water phase comprises liquid crystals.

17. A container comprising a spray nozzle and a sprayable personal care or cosmetic product comprising an oil in water emulsion comprising an emulsifier system for the oil which comprises at least one branched alkoxylated non-ionic surfactant and 0.5 to 10% by weight of the emulsion of at least one surfactant capable of forming liquid crystals in water, wherein the water phase comprises liquid crystals.

18. The use of a surfactant composition comprising at least one branched alkoxylated non-ionic surfactant and at least one surfactant capable of forming liquid crystals in water to stabilize an oil in water emulsion, wherein the water phase comprises liquid crystals and the amount of surfactant capable of forming liquid crystals is 0.5 to 10% by weight of the emulsion.

## Patentansprüche

1. Öl in Wasser Emulsion, umfassend ein Emulgatorsystem für das Öl, welches wenigstens einen verzweigten, alkoxylierten, nicht-ionischen, grenzflächenaktiven Stoff und 0,5 bis 10 Gew.-% wenigstens eines grenzflächenaktiven Stoffs, der fähig zum Bilden von Flüssigkristallen in Wasser ist, umfasst, wobei die Wasserphase Flüssigkristalle umfasst.

2. Emulsion nach Anspruch 1, wobei der verzweigte, alkoxylierte, nicht-ionische, grenzflächenaktive Stoff eine verzweigte Alkylgruppe umfasst, die Kohlenstoffatome in dem Bereich von 12 bis 20 umfasst.

3. Emulsion nach Anspruch 2, wobei die verzweigte Alkylgruppe Seitenverzweigungen in dem Bereich von 0,2 bis 3 umfasst.

4. Emulsion nach Anspruch 3, wobei die seitenverzweigten Gruppen Alkylgruppen darstellen, die Kohlenstoffatome in dem Bereich von 2 bis 9 umfassen.

5. Emulsion nach einem der der vorhergehenden Ansprüche, wobei die Alkoxylatgruppe eine homopolymere Polyoxyethylenkette darstellt, die Ethylenoxid-Gruppen in dem Bereich von 15 bis 25 enthält.

6. Emulsion nach einem der vorhergehenden Ansprüche, wobei der verzweigte, alkoxylierte, nicht-ionische, grenzflächenaktive Stoff ein primäres Alkohol-Alkoxylat umfasst.

7. Emulsion nach Anspruch 6, wobei das primäre Alkohol-Alkoxylat von der Formel ist:
**CH₃-(CH₂)_{q}-[CH₃-(CH₂)ᵣ]-CH-(CH₂)ₚ-O-(CₘH₂ₘO)ₙ-H** **(I)**
wobei
q und r jeweils unabhängig von 0 bis 13 sind, bevorzugt 1 bis 15, stärker bevorzugt 2 bis 13; und p 1 oder 2 ist;
so dass q+r+p in dem Bereich von 9 bis 17 liegt, bevorzugt 10 bis 16, stärker bevorzugt 11 bis 15; und (CₘH₂ₘO)ₙ eine Polyoxyalkylengruppe darstellt, wobei m 2, 3 oder 4 ist, und n in dem Bereich von 5 bis 40 liegt.

8. Emulsion nach einem der vorhergehenden Ansprüche, umfassend 0,5 bis 10 Gew.-% des verzweigten, alkoxylierten, nicht-ionischen, grenzflächenaktiven Stoffs.

9. Emulsion nach einem der vorhergehenden Ansprüche, wobei der grenzflächenaktive Stoff, der fähig zum Bilden von Flüssigkristallen in Wasser ist, einen nicht-alkoxylierten Polyolester umfasst.

10. Emulsion nach Anspruch 9, wobei das Polyol ein Anhydro-Saccharid darstellt und/oder der Ester von einer Fettsäure, die Kohlenstoffatome in dem Bereich von 12 bis 22 umfasst, abgeleitet ist.

11. Emulsion nach einem der Ansprüche 10 und 11, wobei der grenzflächenaktive Stoff, der fähig zum Bilden von Flüssigkristallen in Wasser ist, zusätzlich einen von einem Saccharid abgeleiteten Polyolester umfasst.

12. Emulsion nach Anspruch 11, wobei das Saccharid Sucrose oder Sorbitol ist, und/oder der Ester von einer Fettsäure, die Kohlenstoffatome in einem Bereich von 8 bis 18 umfasst, abgeleitet ist.

13. Emulsion nach einem der vorhergehenden Ansprüche, wobei der grenzflächenaktive Stoff, der fähig zum Bilden von Flüssigkristallen in Wasser ist, eine Mischung eines Sorbitanesters und eines Sucroseesters oder eines Sorbitolesters umfasst.

14. Emulsion nach Anspruch 13, wobei der grenzflächenaktive Stoff, der fähig zum Bilden von Flüssigkristallen in Wasser ist, eine Mischung aus Sorbitanstearat und Sucrosecocoat oder Sorbitollaurat umfasst.

15. Emulsion nach einem der vorhergehenden Ansprüche, wobei der HLB Wert (i) des verzweigten alkoxylierten, nicht-ionischen, grenzflächenaktiven Stoffs in dem Bereich von 13 bis 18 liegt, (ii) des grenzflächenaktives Stoffs, der fähig zum Bilden von Flüssigkristallen in Wasser ist, in dem Bereich von 4 bis 8 liegt, und (iii) der Grenzflächenaktivstoff-Gesamtzusammensetzung in dem Bereich von 8 bis 12 liegt.

16. Körperpflege- oder Kosmetikprodukt, umfassend eine Öl in Wasser Emulsion, die ein Emulgatorsystem für das Öl umfasst, welches wenigstens einen verzweigten, alkoxylierten, nicht-ionischen, grenzflächenaktiven Stoff und 0,5 bis 10 Gew.-% der Emulsion wenigstens eines grenzflächenaktiven Stoffs, der fähig zum Bilden von Flüssigkristallen in Wasser ist, umfasst, wobei die Wasserphase Flüssigkristalle umfasst.

17. Behälter, umfassend eine Sprühdüse und ein sprühbares Körperpflege- oder Kosmetikprodukt, umfassend eine Öl in Wasser Emulsion, die ein Emulgatorsystem für das Öl umfasst, welches wenigstens einen verzweigten, alkoxylierten, nicht-ionischen, grenzflächenaktiven Stoff und 0,5 bis 10 Gew.-% der Emulsion wenigstens eines grenzflächenaktiven Stoffs, der fähig zum Bilden von Flüssigkristallen in Wasser ist, umfasst, wobei die Wasserphase Flüssigkristalle umfasst.

18. Verwendung einer Grenzflächenaktivstoff-Zusammensetzung, umfassend wenigstens einen verzweigten, alkoxylierten, nicht-ionischen, grenzflächenaktiven Stoff und wenigstens einen grenzflächenaktiven Stoff, der fähig zum Bilden von Flüssigkristallen in Wasser ist, um eine Öl in Wasser Emulsion zu stabilisieren, wobei die Wasserphase Flüssigkristalle umfasst und die Menge an grenzflächenaktivem Stoff, der fähig zum Bilden von Flüssigkristallen ist, 0,5 bis 10 Gew.-% der Emulsion beträgt.

## Revendications

1. Emulsion huile-dans-eau comprenant un système émulsifiant pour l'huile, qui comprend au moins un tensioactif non ionique alcoxylé ramifié et 0,5 à 10 % en poids d'au moins un tensioactif capable de former des cristaux liquides dans de l'eau, dans laquelle la phase aqueuse comprend des cristaux liquides.

2. Emulsion selon la revendication 1, dans laquelle le tensioactif non ionique alcoxylé ramifié comprend un groupe alkyle ramifié comprenant de 12 à 20 atomes de carbone.

3. Emulsion selon la revendication 2, dans laquelle le groupe alkyle ramifié comprend 0,2 à 3 chaînes latérales.

4. Emulsion selon la revendication 3, dans laquelle les groupes à chaînes latérales sont des groupes alkyles comprenant de 2 à 9 atomes de carbone.

5. Emulsion selon l'une quelconque des revendications précédentes, dans laquelle le groupe alcoxylate est une chaîne polyoxyéthylénée homopolymère contenant de 15 à 25 résidus d'oxyde d'éthylène.

6. Emulsion selon l'une quelconque des revendications précédentes, dans laquelle le tensioactif non ionique alcoxylé et ramifié comprend un alcoxylate d'alcoolate primaire.

7. Emulsion selon la revendication 6, dans laquelle l'alcoxylate d'alcool primaire est de formule :
CH₃-(CH₂)_{q}-[CH₃-(CH₂)ᵣ]-CH-(CH₂)ₚ-O-(CₘH₂ₘO)ₙ-H (I)
dans laquelle
q et r représentent, chacun indépendamment, un nombre compris entre 0 et 13, de préférence 1 et 15, plus préférentiellement 2 et 13 ; et p est le nombre 1 ou 2 ;
de sorte que la somme q+r+p soit située dans l'intervalle allant de 9 à 17, de préférence de 10 à 16, plus préférentiellement de 11 à 15 ; et
(CₘH₂ₘO)ₙ est un groupe polyoxyalkyléné où m est le nombre 2, 3 ou 4, et n est un nombre compris entre 5 et 40.

8. Emulsion selon l'une quelconque des revendications précédentes, comprenant 0,5 à 10 % en poids de tensioactif non ionique alcoxylé et ramifié.

9. Emulsion selon l'une quelconque des revendications précédentes, dans laquelle le tensioactif capable de former des cristaux liquides dans de l'eau comprend un ester de polyol non alcoxylé.

10. Emulsion selon la revendication 9, dans laquelle le polyol est un anhydrosaccharide et/ou l'ester est dérivé d'un acide gras comprenant de 12 à 22 atomes de carbone.

11. Emulsion selon l'une ou l'autre des revendications 9 et 10, dans laquelle le tensioactif capable de former des cristaux liquides dans de l'eau comprend en plus un ester de polyol dérivé d'un saccharide.

12. Emulsion selon la revendication 11, dans lequel le saccharide est le saccharose ou le sorbitol, et/ou l'ester est dérivé d'un acide gras comprenant de 8 à 18 atomes de carbone.

13. Emulsion selon l'une quelconque des revendications précédentes, dans laquelle le tensioactif capable de former des cristaux liquides dans de l'eau comprend un mélange d'un ester de sorbitane et d'un ester de saccharose ou d'un ester de sorbitol.

14. Emulsion selon la revendication 13, dans laquelle le tensioactif capable de former des cristaux liquides dans de l'eau comprend un mélange de stéarate de sorbitane et de cocoate de saccharose ou de laurate de sorbitol.

15. Emulsion selon l'une quelconque des revendications précédentes, dans laquelle la valeur HLB (i) du tensioactif alcoxylé ramifié est située dans l'intervalle allant de 13 à 18, (ii) du tensioactif capable de former des cristaux liquides dans de l'eau est située dans l'intervalle allant de 4 à 8, et (iii) de la composition tensioactive totale est située dans l'intervalle allant de 8 à 12.

16. Produit de soin personnel ou cosmétique comprenant une émulsion huile-dans-eau, contenant un système émulsifiant pour l'huile qui comprend au moins un tensioactif non ionique alcoxylé ramifié et 0,5 à 10 % en poids de l'émulsion d'au moins un tensioactif capable de former des cristaux liquides dans de l'eau, dans lequel la phase aqueuse comprend des cristaux liquides.

17. Récipient comprenant une buse de pulvérisation et un produit de soin personnel ou cosmétique pulvérisable comprenant une émulsion huile-dans-eau, contenant un système émulsifiant pour l'huile qui comprend au moins un tensioactif non ionique alcoxylé ramifié et 0,5 à 10 % en poids de l'émulsion d'au moins un tensioactif capable de former des cristaux liquides dans de l'eau, dans lequel la phase aqueuse comprend des cristaux liquides.

18. Utilisation d'une composition tensioactive comprenant au moins un tensioactif non ionique alcoxylé ramifié et au moins un tensioactif capable de former des cristaux liquides dans de l'eau pour stabiliser une émulsion huile-dans-eau, dans laquelle la phase aqueuse comprend des cristaux liquides et la quantité de tensioactif capable de former des cristaux liquides constitue 0,5 à 10 % en poids de l'émulsion.
